(19) Europäisches Patentamt
European Patent Office
Office européen des brevets

(11) Publication number: 0 007 191
B1

# (12) EUROPEAN PATENT SPECIFICATION

(45) Date of publication of patent specification: 01.12.82

(51) Int. Cl.³: A 61 K 7/38, C 07 F 5/06

(21) Application number: 79301204.8

(22) Date of filing: 21.06.79

## (54) Antiperspirant basic aluminium chloride/polyhydroxy compound complex.

(30) Priority: 23.06.78 GB 2775478

(43) Date of publication of application:
23.01.80 Bulletin 80/2

(45) Publication of the grant of the patent:
01.12.82 Bulletin 82/48

(84) Designated Contracting States:
DE FR GB

(56) References cited:
FR - A - 2 259 587
FR - A - 2 278 319
US - A - 3 359 169
US - A - 3 420 932
US - A - 3 472 929
US - A - 3 507 896

(73) Proprietor: UNILEVER PLC
Unilever House Blackfriars P O Box 68
London EC4P 4BQ (GB)
(84) GB

(73) Proprietor: UNILEVER NV
Burgemeester s'Jacobplein 1 P.O. Box 760
NL-3000 DK Rotterdam (NL)
(84) DE FR

(72) Inventor: Goslins, Keith
Flat 5, 50/52 Mont Ararat Road
Richmond, Surrey (GB)
Inventor: Sime, Wilma Jean
132 Ewell by-pass
Ewell, Surrey (GB)
Inventor: Mulley, Victor John
12 Selsdon Avenue Woodley
Reading, Berkshire (GB)
Inventor: Baldock, Michael John
17 Shrewsbury Walk
Isleworth, Middlesex (GB)

(74) Representative: Doucy, Robert Henry et al,
Unilever PLC, Patent Division PO Box 31 Salisbury
Square House Salisbury Square
London EC4 4AN (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 007 191 B1

Courier Press, Leamington Spa, England.

# Antiperspirant basic aluminium chloride/polyhydroxy compound complex

The invention relates to antiperspirants.

In our copending German Application P 27 00 711.0 we have described basic aluminium chloride, bromide, iodide and nitrate compounds having enhanced antiperspirant activity. Although basic aluminium compounds are well known to have antiperspirant activity it has been shown in the copending application referred to that an enhancement in the antiperspirant activity of basic aluminium compounds having an aluminium to chloride, bromide, iodide or nitrate molar ratio of from 1.3 to 6.5:1 can be obtained by prolonged heating of aqueous solutions of said compounds under certain conditions leading to the formation of higher polymeric species having a size above 100 Ångstroms. The antiperspirant active compounds having enhanced activity are defined in the said copending application as those which in aqueous solution there is at least 2% by weight of the total aluminium contained in polymeric species having a size greater than 100 Ångstroms. The improved antiperspirant material may be obtained in powdered form by drying, e.g. spray drying, the aqueous solution. This powder, however, has the disadvantage that it is not soluble in alcohol.

U.S. Patents Nos. 3,359,169 and 3,420,932 describe the preparation of alcohol-soluble complexes of a basic aluminium chloride and a polyhydroxy compound, e.g. propylene glycol. In U.S. Patent No. 3,420,932 the polyhydroxy compound is defined as one having at least 2 carbon atoms each of which is linked to an hydroxy group. A complex of aluminium chlorhydrate and propylene glycol is commercially available under the trade mark "Rehydrol" from the Reheis Chemical Company, Division of Armour Pharmaceutical Company.

This invention has for an object to provide novel alcohol-soluble basic aluminium compounds having good antiperspirant activity.

According to the invention there is provided a particulate basic aluminium chloride/polyhydroxy compound complex of the empirical formula:

$$Al_2(OH)_{6-a}Cl_a(H_2O)_xR_y$$

where R is a polyhydroxy compound having at least 2 carbon atoms each of which is linked to a hydroxy group; *a* has a value of from 0.8 to 4.0, preferably 0.95 to 2.0; *x* has a value of from 0.5 to 2.5, preferably 0.75 to 2.0; and *y* has a value of from 0.2 to 5.0, preferably 0.3 to 2.0; which complex has less than 10% of the total aluminium contained in polymeric species having a size exceeding 100 Ångstroms and which has such size distribution of polymeric species that in the size exclusion chromatographic procedure described herein there is eluted a fraction between the relative retention times of 0.76 and 0.82 in which fraction there is contained at least 15% of the total aluminium of the compound. For convenience, such fraction is referred to hereinafter as the Band III fraction. The amount of aluminium contained in the Band III fraction is preferably at least 20%, more preferable at least 25%, and may even exceed 80%, of the total aluminium.

Characterisation of materials containing species differing in size by means of size exclusion chromatography is generally known. The size exclusion chromatographic procedure for characterising the basic aluminium compounds of this invention will now be described.

The analytical procedure is performed on a stainless steel column of dimensions 30 cm high and of 7 mm internal diameter packed with porous silica of nominal particle size 5 microns and pore size of 60 Ångstroms, which silica has been deactivated by silylation to eliminate adsorption in size exclusion separations. A suitable silica is that available commercially as LiChrosorb RP-2. The silica employed by the Applicants in deriving analytical data given herein had a cumulative undersize particle size distribution by weight of 10% less than 5 microns, 50% less than 6 microns and 90% less than 7 microns.

The column is fitted at the bottom with a zero dead volume fitting containing a 2 micron mesh stainless steel bed support. The silica is packed into the column by the high pressure slurry method (see Practical High Performance Liquid Chromatography, Edited by C. F. Simpson, 1976, Appendex II), using methanol:water (90:10) containing 1% sodium acetate as the packing medium.

After packing, the column is capped with another zero dead volume fitting containing a 2 micron stainless steel mesh. The packed column is then eluted with 200 ml of methanol at a flow rate of about 10 ml/min, using a high presusre pump, to consolidate the bed and wash out the packing medium. The bed is topped up, if necessary, with a thick slurry of the packing in methanol followed by reconsolidation.

A differential refractive index monitor (e.g. Waters R401) is used to detect sample fractions as they are eluted. It is linked to a pen recorder to provide a chromatogram and to an integrator (e.g. Infotronics CRS 309) which measures the elution times of the fractions and the relative chromatographic band areas. The integrator is required to measure areas of bands not resolved to the baseline by dropping perpendiculars from the lowest point of the valleys separating the bands to the baseline.

The column packing should be tested according to the procedure of Bristow & Knox

(Chromatographia, Volume 10, No. 6, June 1977, pp 279—89) for reverse phase materials and should generate at least 20,000 plates/metre for the test component phenetole.

To prepare test solutions of the materials for analysis those already in solution are diluted, if necessary, with deionized water to give 2 g of a solution containing 2.5% by weight aluminium and dispersed by treatment with a sonic probe for 2 minutes. Solid materials (e.g. spray dried powders) are dissolved in deionized water to give 2 g of a solution containing 2.5% by weight aluminium and dispersed by treatment with a sonic probe for 2 minutes. The solutions prepared in this way are filtered through a 25 mm diameter membrane having a pore size of 0.025 micrometres to give the test solutions. The preparation of a test solution is carried out immediately prior to application of a sample thereof to the column.

A sample of the test solution containing about 4 micromoles of aluminium is applied to the top of the column by means of a precision microlitre syringe and a sample injection port. The sample is eluted with a $1 \times 10^{-2}$ M aqueous nitric acid solution at a flow rate of 1.0 ml/min using a high pressure pump. The eluent is maintained at a temperature of 22—23°C.

Eluted fractions of a test sample are characterized by means of the ratio of their retention times to the retention time of the totally included species. The totally included species arises from hydrochloric acid (which is present in solutions of basic aluminium chlorides) as can be shown by comparison of its retention time with that of a sample of hydrochloric acid.

In aqueous solution the basic aluminium chloride/polyhydroxy compound complex dissociates to give the polyhydroxy compound and a solution of basic aluminium chloride. In the size exclusion chromatography the polyhydroxy compound separates by a reverse phase process and is eluted from the column at a relative retention time greater than 1.

Using columns satisfying the above description and employing a standard solution of a basic aluminium chloride prepared as described below, the Applicants have obtained separation into four aluminium-containing fractions having relative retention times within the ranges indicated.

| | Band I | Band II | Band III | Band IV |
|---|---|---|---|---|
| Relative Retention Time Range | 0.62—0.70 | 0.71—0.75 | 0.76—0.82 | 0.83—0.97 |

The standard basic aluminium chloride solution is prepared as a solution containing 12.5% by weight aluminium from 19.1 g of aluminium chloride hexahydrate, 10.5 g of 99.9% pure aluminium wire (0.76 mm diameter, cut in approximately 1 cm lengths and degreased by washing in acetone) and 70.4 g of deionised water. The mixture is stirred and heated at 80—90°C under a reflux condenser until all of the aluminium is dissolved. Any traces of insoluble solids are removed by filtration to give a clear solution.

When this material is analysed by the size exclusion chromatographic procedure described herein, there are obtained the following four fractions having typical relative retention times and chromatographic band areas expressed as percentages of the total chromatographic band area representing aluminium-containing material.

| | Band I | Band II | Band III | Band IV |
|---|---|---|---|---|
| Relative Retention Time | 0.65 | 0.73 | 0.79 | 0.91 |
| Band Area % of total aluminium band area | 39 | 51 | 4 | 6 |

The standard solutions contained 0% aluminium as polymers greater than 100 Ångstroms in effective diameter.

It will be appreciated by those skilled in the art that mechanisms of separation other than the principal mechanism of size exclusion may play a part in this type of chromatography. Examples of the processes would be adsorption effects and hydrodynamic effects. Thus although it is possible for a given column and constant operating conditions to lead to invariable relative retention times, minor variations in particle size range and pore size distribution of the column packing material may lead to slight differences in relative retention times.

Quantitatively, the amount of aluminium in the Band III fraction expressed as a percentage of the total aluminium of the compound under test is readily determined from the area of its band on the chromatogram. This percentage is derived from the expression

$$\% \text{ Aluminium} = (100\text{-}A) \times \frac{\text{Area of band corresponding to Band III fraction}}{\text{Sum of the areas of the bands corresponding to the aluminium containing fractions}}$$

where A is the percentage of the total aluminium which is contained in polymers greater than 100 Angstroms and is determined by the method described hereinafter.

In experiments performed by the Applicants using certain samples of test materials, the complete elution of all the applied aluminium in a sample was checked by direct analysis of another sample of the same volume by plasma emission spectrophotometry. The correlation between band area percentage and aluminium percentage was also verified by direct analysis. The fractions were collected as they emerged from the refractive index monitor and their individual aluminium contents measured also by plasma emission spectrophotometry.

While the polyhydroxy compound is preferably propylene glycol, other compounds such as those referred to in U.S. Patent No. 3,420,932 may be used, e.g. 1,1,1-trimethylol propane, 1,3-butylene glycol, glycerine, 2-methyl-2,4-pentane diol, neopentyl glycol, polyethylene glycol (molecular weight 400).

Preferred products of the invention having especially good solubility in alcohol are those which have a value of *y* from 0.5 to 5 and contain less than 4%, more preferably 0% of the total aluminium in polymers having a size exceeding 100 Angstroms.

The basic aluminium chloride/polyhydroxy compound complexes of this invention may be made in various ways. We have found that enhanced activity is linked with increase in the percentage of the aluminium in the Band III fraction. Such increase may be brought about by heating an aqueous solution of a basic aluminium chloride of the empirical formula $Al_2(OH)_{6-a}Cl_a$ where *a* has the above meaning under certain conditions of concentration, temperature, and time as generally described in our concurrently filed Application No. 79301203.0, Publication No. 0006739 corresponding to British Application No. 27755/78. The aluminium concentration of the solution may be from 2.5% to about 8.5% by weight, lower concentrations favouring more rapid production of species represented by the Band III fraction. The rate of production of such species is also favoured by increase in temperature. However, it is required to avoid or limit the production of polymers exceeding 100 Angstroms in size and to achieve this it is desired to use relatively concentrated solutions and/or basic aluminium chlorides having a relatively low Al:Cl molar ratio. The production of such higher polymers is also favoured by the use of higher temperatures and longer heating times. Taking the above factors into account it can therefore be stated as a generalisation that it is preferable to employ solutions of aluminium concentration of 3.7 to 6.8% by weight and that should solutions of lower concentration be used, e.g. a solution having an aluminium concentration of 2.5%, then the Al:Cl molar ratio is preferably less than 1.95. Heating times of from 0.5 to 20 days are preferred.

It is the heating process referred to in the preceding paragraph that leads to the improved antiperspirant activity of the basic aluminium chloride complex. We have found that powdered material having good alcohol solubility can be obtained either by carrying out the heating process in the presence of the polyhydroxy compound and subsequently drying the aqueous solution to produce a dry powdered product, or by adding the polyhydroxy compound after the heating process but prior to the drying stage. The drying may be effected by known procedures (see U.S. Patent No. 3,420,932), preferably by spray drying at a temperature between 80 and 320°C.

The test method for testing antiperspirant efficacy referred to in the Examples given herein will now be described. The test method depends on subjecting human volunteers to thermal stress and gravimetric determination of axillar sweat.

## Test method

Subjects

A panel of up to 54 women who use no antiperspirant for the 14 days before the test.

Hot room

Temperatures 37°C±1°C, relative humidity approximately 35%.

Test product

20% w/w solution in ethanol of test product.

Control product

20% w/w solution in ethanol of a commercially available aluminium chlorhydrate/propylene glycol complex.

Method of application

Approximately 0.5 g of solution was applied to each axilla with a pump-spray applicator.

Sweat collection

Absorbent cotton pads are used to collect the sweat. On entering the hot room each subject has a pad placed in each of her axillae. After 40 minutes these are removed and rejected. Sweat is then collected for two consecutive periods of 20 minutes, fresh tared pads being used for each collection, and sweat weight determined.

Test design

Subjects attend daily for 3 consecutive days. They receive one treatment with the products each day. On the third day the treatment is immediately followed by a hot room sitting and sweat collection.

Analysis of data

The statistical treatment includes an analysis of variance which allows for subject, side and product effects. The efficacy is calculated from the geometric mean weight of sweat collected from the axiallae treated with each product.

$$\% \text{ reduction}=100\frac{(C-T)}{C}$$

where C is the geometric mean sweat weight from the axillae treated with the control product and T is the geometric mean sweat from the axillae treated with the test product. The % reduction is usually calculated for each day separately and for the entire test. Significance is calculated by applying Student's t-test to the logarithmically transformed weights.

Determination of percentage aluminium in polymeric species having a size greater than 100 Ångstroms

For this purpose there was used a 1.2 m×6.0 mm column packed with sperical porous silica beads of particle size 75—125 microns, and of surface area 350—500 $m^2/g$, and having a maximum pore size of 100 Ångstroms. The silica employed, available commercially as Porasil AX, had been deactivated to eliminate adsorption in molecular size separations. The use of Porasil silica beads as a column packing in chromatography is referred to in "Gel Permeation Chromatography" by K. H. Altgelt and L. Segal, 1971, pages 16—18. The silica was conditioned before use by passage of a single large sample (e.g. 0.2 ml of a 5% w/w solution) of a heat-treated aluminium chlorhydrate. Samples to be tested were made up in deionized water to approximately 0.2 M aluminium and thoroughly dispersed by treatment (4 minutes) with a sonic probe. About 0.2 ml samples of approximately 0.2 M aluminium solutions were applied to the column by a sample loop system and eluted with a $10^{-2}$ M aqueous nitric acid solution using a peristaltic pump. A differential refractive index monitor linked to a pen recorder was used to detect fractions as they were eluted. These fractions were collected and analysed for aluminium by atomic adsorption. Complete elution of all aluminium applied in each sample was checked by direct analysis of another sample of the same volume. The percentage of the total aluminium which appeared in the fraction eluted at the void volume of the column was considered as that deriving from polymeric material of a size greater than 100 Ångstroms in effective diameter.

The following Examples illustrate the invention.

Example 1

200 g of aluminium chlorhydrate (Al:Cl molar ratio of 1.93) and 100 g of propylene glycol (the Al:propylene glycol molar ratio of the mixture being 1.52) were dissolved in deionised water to give a total weight of 1,000 g. This solution was placed in 25 ml Pyrex screw cap tubes equipped with polytetrafluoroethylene washers and placed in an oven preheated to 120°C (the word Pyrex is a Trade Mark). After an initial warm up period of 30 minutes the tubes were heated for 4 hours. They were then removed and cooled to ambient temperature. The resulting solution was spray dried in a co-current spray drier with an inlet temperature of 250°C and an outlet temperature of 90°C, to give a white powder soluble in ethanol to give a 20% w/w solution. The material was tested for antiperspirancy as a 20% w/w solution in ethanol using the test method described above with a panel of 24 subjects, using as control a 20% solution in ethanol of an aluminium chlorhydrate/propylene glycol complex (having an Al:Cl molar ratio of 2.0 and an Al:propylene glycol molar ratio of 2.3) and commercially available under the trade name "Rehydrol". The test product produced a reduction of 10% in the sweat collected, compared with the control product, the difference being statistically significant at the 35% level.

Analysis of the spray dried powder showed that it has the following empirical formula:

$$Al_2(OH)_{4.97}Cl_{1.03}(H_2O)_{1.39}(\text{propylene glycol})_{0.65}$$

The complex was shown to contain 1% by weight of its aluminium in polymers exceeding 100 Ångstroms in size. The amount of aluminium in the Band III fraction was 30% of the total aluminium.

The complex used as control material on analysis showed no polymers above 100 Ångstroms in aqueous solution and about 7% of the aluminium in the Band III fraction.

Example 2

200 g of a commercially available aluminium chlorhydrate having an Al:Cl molar ratio of 2.0 were dissolved in deionised water to give a total weight of 1,000 g. This solution was placed in 25 ml Pyrex screw cap tubes equipped with polytetrafluoroethylene washers and placed in an oven preheated to

120°C. After an initial warm up period of 30 minutes the tubes were heated for 4 hours. They were then removed and cooled to ambient temperature. 120 g of propylene glycol were added to the solution which was then spray dried as in Example 1. The resulting white powder was soluble in ethanol to give a 40% w/w solution. The material was tested for antiperspirancy as a 20% w/w solution in ethanol using the test method and control as in Example 1 with a panel of 24 subjects and produced a reduction of 19% in the sweat collected, the difference being statistically significant at the 5% level.

Analysis of the spray dried powder showed that it had the following empirical formula:

$$Al_2(OH)_{5.02}Cl_{0.98}(H_2O)_{1.57}(\text{propylene glycol})_{0.59}$$

The complex was shown to contain 0% of the aluminium in polymers exceeding 100 Ångstroms in size. The amount of aluminium in the Band III fraction was 31% of the total aluminium.

Example 3

Aluminium chlorhydrate having an Al:Cl molar ratio of 2.0, aluminium chloride hexahydrate and deionised water were mixed to give a solution with an Al:Cl molar ratio of 1.6 and having an aluminium concentration of 0.95 moles per litre. The solution was heated at 120°C for 24 hours. After cooling the solution, an amount of propylene glycol corresponding to one mole per mole of aluminium was added. The solution was then spray dried as in Example 1. The spray dried powder was soluble in ethanol to give a 25% solution.

The complex was shown to contain 0% of the aluminium in polymers exceeding 100 Ångstroms in size. The amount of aluminium in the Band III fraction was 29% of the total aluminium.

Example 4

The procedure of Example 3 was repeated save that the amount of aluminium chloride was such as to give an Al:Cl molar ratio of 1.14. The spray dried product was soluble in ethanol to give a 25% solution.

The complex was shown to contain 0% of the aluminium in polymers exceeding 100 Angstroms in size. The amount of aluminium in the Band III fraction was 26% of the total aluminium.

**Claims**

1. A particulate basic aluminium chloride/polyhydroxy compound complex of the empirical formula:

$$Al_2(OH)_{6-a}Cl_a(H_2O)_xR_y$$

where R is a polyhydroxy compound having at least 2 carbon atoms each of which is linked to a hydroxy group; *a* has a value of from 0.8 to 4.0, *x* has a value of from 0.5 to 2.5, and *y* has a value of from 0.2 to 5.0, characterized in that the complex has at least 15% of the total aluminium in the Band III fraction as measured by size exclusion chromatography and less than 10% of the total aluminium contained in polymers having a size exceeding 100 Ångstroms.

2. A particulate complex as claimed in claim 1, characterized in that the complex has at least 20% of the total aluminium in the Band III fraction.

3. A particulate complex as claimed in claim 2, characterized in that the complex has from 25 to 80% of the total aluminium in the Band III fraction.

4. A particulate complex as claimed in any of the preceding claims, characterized in that the complex has less than 4% of the total aluminium contained in polymers having a size exceeding 100 Ångstroms.

5. A particulate complex as claimed in any of the preceding claims, characterized in that the polyhydroxy compound is propylene glycol.

**Revendications**

1. Un complexe en particules chlorure d'aluminium basique/composé polyhydroxylé répondant à la formule empirique:

$$Al_2(OH)_{6-a}Cl_a(H_2O)_xR_y$$

dans laquelle R est un composé polyhydroxylé contenant au moins 2 atoms de carbone reliés chacun à un groupe hydroxy; *a* a une valeur de 0,8 à 4,0, *x* a une valeur de 0,5 à 2,5 et *y* a une valeur de 0,2 à 5,0, caractérisé en ce que le complexe contient au mouns 15% de l'aluminium total dans la fraction de bande III, la mesure étant faite par chromatographie d'exclusion de dimensions, et moins de 10% de l'aluminium total dans des polymères dont la dimension dépasse 100 Angströms.

2. Un complexe en particules selon la revendication 1, caractérisé en ce qu'il contient au moins 20% de l'aluminium total dans la fraction de bande III.

3. Un complexe en particules selon la revendication 2, caractérisé en ce qu'il contient de 25 à 80% de l'aluminium total dans la fraction de bande III.

4. Un complexe en particules selon l'une quelconque des revendications qui précèdent, caractérisé en ce qu'il contient moins de 4% de l'aluminium total dans des polymères dont la dimension dépasse 100 Angströms.

5. Un complexe en particules selon l'une quelconque des revendications qui précèdent, caractérisé en ce que le composé polyhydroxylé est le propylène-glycol.

**Patentansprüche**

1. Teilchenförmiger, basischer Aluminiumchlorid/Polyhydroxy-Verbindung-Komplex der empirischen Formel

$$Al_2(OH)_{6-a}Cl_a(H_2O)_xR_y,$$

worin R eine Polyhydroxy-Verbindung mit wenigstens 2 Kohlenstoffatomen, deren jedes mit einer Hydroxygruppe verbunden ist, ist, a einen Wert von 0,8 bis 4,0 hat, x einen Wert von 0,5 bis 2,5 hat und y einen Wert von 0,2 bis 5,0 hat, dadurch gekennzeichnet, daß der Komplex wenigstens 15% des gesamten Aluminiums in der Bande III-Fraktion, gemessen durch Größenausschlußchromatographie, und weniger als 10% des gesamten Aluminiums in Polymeren mit einer Größe über 100 Å enthält.

2. Teilchenförmiger Komplex nach Anspruch 1, dadurch gekennzeichnet, daß der Komplex wenigstens 20% des gesamten Aluminiums in der Bande III-Fraktion hat.

3. Teilchenförmiger Komplex nach Anspruch 2, dadurch gekennzeichnet, daß der Komplex 25 bis 80% des gesamten Aluminiums in der Bande III-Fraktion hat.

4. Teilchenförmiger Komplex nach einem der vorhergehenden Ansprüche dadurch gekennzeichnet, daß der Komplex weniger als 4% des gesamten Aluminiums in Polymeren mit einer Größe über 100 Å enthält.

5. Teilchenförmiger Komplex nach einem der vorhergehenden Ansprüche, dadurch gekennzeichnet, daß die Polyhydroxy-Verbindung Propylenglykol ist.